# EUROPEAN PATENT APPLICATION

(11) **EP 2 298 923 A1**
(43) Date of publication of application: **23.03.2011**
(21) Application number: 09170630.9
(22) Date of filing: 18.09.2009
(51) Int. Cl.: C12N 15/82

(54) **Method for the production of recombinant proteins by green microalgae**

(71) Applicant: Université de Liège, 4031 Angleur (BE)
(72) Inventor: Clérisse, Fabienne, 4000 Liège (BE); Dommes, Jacques, 4000 Liège (BE); Gauthier, Carole, 4000 Liège (BE); Versali, Marie-France, 4000 Liège (BE)
(74) Representative: Brants, Johan P.E.

(57) **Abstract**

The present invention relates to a method for the production of recombinant proteins by algal cells, which are fresh water unicellular green microalgae belonging to the order of *Sphaeropleales.* The present invention further provides recombinant algal cells, wherein said algal cells are fresh water unicellular green microalgae belonging to the order of *Sphaeropleales,* and wherein said algal cells are capable of producing a recombinant protein. The invention is also directed to a method for selecting recombinant algal cells. The invention also relates to the use of recombinant algal cells as described herein for producing recombinant proteins.

## Description

### Technical field

The invention is in the field of molecular biology, and relates to recombinant engineering and protein expression. In particular, the invention relates to a method for producing recombinant proteins by fresh water unicellular green microalgae. More specifically, the present invention relates to a method for producing a recombinant protein by a recombinant algal cell of the order of the *Sphaeropleales.* The invention also relates to a recombinant algal cell of the order of the *Sphaeropleales* capable of producing recombinant proteins. The invention further also relates to the use of recombinant algal cells for producing recombinant proteins.

### Background

There is a growing demand for recombinant proteins, and industry is looking for new production systems with the following characteristics: high production capacity at low cost, straight forward to use, able to produce active eukaryotic proteins and GRAS status (absence of animal or human pathogen and of any toxic compound).

Unicellular algae could meet these requirements while providing the market with adequate production capacity. Algae propagate by vegetative replication, lack pollen, and have no potential for gene transfer to food crops. They can easily be grown in containment, again reducing any chance of environmental contamination. Their controlled growth also assures that external contaminants, like pesticides or pollutants, do not contaminate the protein being produced. Algae are eukaryotes, meaning that unlike bacteria, they are efficient at producing complex proteins and have the machinery necessary to fold and assemble multi-component complexes into functional proteins. In addition, green algae are generally regarded as safe (GRAS), posing little risk of viral, prion or bacterial endotoxin contamination. Thus, algae would seem to be an ideal system for biologic production, as long as high levels of protein expression can be achieved, and that the expressed protein can be shown to function in a bioactive manner.

However, the full potential of algae can only be fully developed with an increase in the ability to genetically tailor algae for specific purposes.

Unfortunately, molecular biological manipulation of algal systems has seriously lagged behind other systems. Many of the techniques that have been developed for the introduction of DNA into bacterial, yeast, insect and animal cells cannot be easily adapted to algal systems. There have been some reports in the art of genetic engineering of algal cells. However, there are no universally applicable techniques for transforming, selecting and growing recombinant algae that can be applied in all types of algal systems.

WO 97/39106 for instance discloses the genetic engineering of eukaryotic marine algae, i.e. algae that grow in sea water, i.e. under marine tidal influence. However, the techniques described for engineering marine algae cannot simply be extrapolated or used for fresh water algae, especially in view of the specific growing requirements of marine algae.

The *Sphaeropleales* are an order of green algae that include non-motile unicellular microalgae that have biflagellate zoospores with flagella that are directly opposed in direction. This type of microalgae can be cultivated and shows benefits, including the absence of animal or human pathogens and of any toxic compound when naturally cultured. However, up to date no adequate techniques have been described for the transformation of this type of algal cells in an economically and industrially applicable way.

In view of the above, it is clear that there remains a need in the art for improved methods and tools for genetically engineering of fresh water eukaryotic algae in order to manufacture products, e.g. recombinant proteins of interest by these algae.

### Summary

To meet the growing industrial demand for recombinant proteins the Applicants have developed a new technology using fresh water microalgae as expression hosts. More precisely the technology utilizes unicellular green micro-algae of the order *Sphaeropleales.* It consists in methods, constructs and tools for the genetic transformation of this order of micro-algae, for the construction of vectors for expression of recombinant (homologous or heterologous) proteins in these algal hosts, for the selection of genetically modified algae, as well as the use of the genetically modified algae or subparts of it, and the produced recombinant proteins after complete or partial purification.

The technology described here allows the expression and the production of recombinant proteins in these unicellular microalgae. It can be used to produce proteins for the pharmaceutical, biomedical, diagnostic, cosmetic, agro-food, environmental and biotechnological industries. Target applications are all proteins of interest in whatever sector of industry, including subunit vaccines, antibodies, hormones, therapeutic molecules, diagnostic molecules, enzymes, food proteins. This list of potential applications is not exhaustive.

The technology includes methods and conditions for genetic transformation of unicellular green micro-algae, mainly from the order *Sphaeropleales,* and particularly from the genus *Scenedesmus*; the construction of vectors allowing recombinant (homologous or heterologous) protein expression in these micro-algae; the selection of the genetically modified algae; the utilization of these genetically modified microalgae or subparts of them, as well as of the produced recombinant proteins either purified to homogeneity or partially purified.

The present invention is in particular directed to a method for the production of a recombinant protein by an algal cell, wherein said algal cell is a fresh water unicellular green microalgae belonging to the order of *Sphaeropleales.*

It is unexpected that a method has been established for successfully engineering algal cells of this order of *Sphaeropleales,* especially in view of characteristics of this type of algal cells that complicate genetic engineering. The cell wall of *Sphaeropleales* algae contains an external tri-lamellar layer made of a polymer called "sporopollenin-like", also known as algaenan. This polymer is not soluble and is very resistant to hydrolysis. In *Sphaeropleales,* this polymer is found in many genera and species including but not limited to *Pediastrum sp., Coelastrum sp,* such as for instance *C*. *microporum, Scotiella sp.,* such as for instance *S*. *chlorelloidea, Ankistrodesmus brunii, Enallax coelastroides, Botryococcus braunii,* and *Scenedesmus sp.,* such as for instance *S*. *obliquus* or *S*. *quadricauda,* where it may represent even up to 33-44% of the cell wall. Moreover, in these genera and species, this polymer is present in vegetative cells which are used for genetic transformation. The presence of this polymer is believed to be responsible for the high physical and chemical resistance and of the impermeability of the cell wall of algal cells of this order, such as e.g. *Scenedesmus* sp. This feature is expected to make genetic transformation of whole cells quite problematic. Nevertheless, despite these prior art teachings, the Applicants have now unexpectedly been able to establish a reproducible method for genetically engineering algal cells of the above *Sphaeropleales* order, even if "sporopollenin-like" material is present in the (vegetative) algal cells which are used for genetic transformation.

More in particular, in a first aspect, the invention relates to a method for the production of a recombinant protein by an algal cell, wherein said algal cell is of the order of *Sphaeropleales,* capable of producing said recombinant protein, comprising the steps of:
a) preparing an expression vector comprising a recombinant nucleic acid comprising a promoter operably linked to at least one protein coding sequence encoding said recombinant protein,
b) transforming said algal cell with said expression vector;
c) selecting transformed algal cells;
d) culturing said selected algal cell in a suitable culture medium under conditions permitting the production of said recombinant protein,
e) recovering said recombinant protein from said culture medium, and
f) optionally purifying said recovered protein.

In a preferred embodiment said algal cell is selected from the genus *Scenedesmus.* In a preferred example, said algal cell is *Scenedesmus obliquus.*

In an embodiment said recombinant protein is a heterologous protein originating from an organism other than said microalgae and preferably from an organism selected from the group comprising humans, animals, plants, micro-organisms, and other algae or microalgae. In another embodiment, said recombinant protein is selected from the group comprising vaccines, antibodies, antigens, hormones, enzymes, therapeutic molecules, diagnostic molecules, and food proteins.

In yet another embodiment said expression vector further comprises a selection marker gene operably linked to a promoter. Said selection marker gene may be selected from the group comprising the *aphVIII* gene from *Streptomyces rimosus*; the *nptII* gene from *Escherichia coli;* the *aph7*" or *hph* gene from *Streptomyces hygroscopes*; the *hpt* gene from *E*. *coli;* the *ble* gene from *Streptoalloteichus hindustanus;* the *ble* gene from *Staphylococcus aureus;* the *bar* gene from *S*. *hygroscopes*; and the *pat* gene from *S*. *viridochromogenes.*

In yet another embodiment, promoter used in the present expression vector may be selected from the group comprising *Chlamydomonas reinhardtii psaD, C*. *reinhardtii β-tubulin, C*. *reinhardtii rbcS2, C*. *reinhardtii hsp70A, C*. *reinhardtii rbcS2-hsp70A* hybrid, and *CaMV 35S* or fragments thereof. The *psaD,* β*-tubulin, rbcS2, hsp70A, and rbcS2 promoters are obtained from Chlamydomonas reinhardtii.*

In another embodiment said protein coding sequence is fused to a nucleotide sequence coding for a secretion signal peptide. In yet another embodiment said protein coding sequence is fused to a nucleotide sequence coding for a tag.

In another embodiment, said recombinant nucleic acid includes one or more introns located between the promoter and the protein coding sequence, or located inside the protein coding sequence.

In another embodiment a method is provided wherein transformation is done by electroporation of algal cells. In a preferred embodiment, the invention provides for the electroporation of whole algal cells. The Applicants have unexpectedly shown that transformation rates obtained by electroporation of algal cells of the order of the *Sphaeropleales,* and in particular of the genus *Scenedesmus,* is much higher than those reported for algal cells of other orders, including for instance *Chlamydomonas* sp. *Chlorella* sp, or *Dunaliella* species.

The Applicants have also established a reproducible technique for rapidly screening or selecting transformed algal cells. In particular, in yet another embodiment of the present method, step c) of selecting transformed algal cells comprises the steps of:
i) preparing an aqueous extract of transformed algal cells by transferring said transformed algal cells in water,
ii) optionally incubating said aqueous extract prepared in step i) at a temperature lower than -20°C for at least 1 hour;
iii) incubating the aqueous extract prepared in step i) or in step ii) at a temperature of at least 95°C for at least 5 minutes; and
iv) subjecting an amount of said aqueous extract to a PCR analysis.

In another aspect, the invention provides a recombinant algal cell, wherein said recombinant algal cell is a fresh water unicellular green microalgae belonging to the order of *Sphaeropleales.* In particular, the invention relates to a recombinant algal cell wherein said algal cell is of the order of *Sphaeropleales* which is transformed with an expression vector as defined herein. In a preferred embodiment, the invention provides a recombinant algal cell as defined herein which is selected from the genus *Scenedesmus,* and which in a preferred example is *Scenedesmus obliquus.*

In another embodiment, the invention provides a recombinant algal cell as defined herein that is obtained or obtainable with by carrying out a method as described herein.

In another aspect, the invention is directed to the use of a recombinant algal cell according to the invention for the production of a recombinant protein. Preferably said recombinant protein is a protein as defined herein, and for instance a heterologous protein herein originating from an organism as defined other than said algal cell.

In an embodiment, the invention relates to the use of a recombinant algal cell as given herein for the production of a recombinant protein, preferably a recombinant protein as defined herein, suitable for being used in a pharmaceutical, biomedical, diagnostic, cosmetic, agro-food, environmental and/or biotechnological application.

In a preferred embodiment, the invention relates to the use of a recombinant algal cell as provided herein for the production of therapeutic proteins selected from the group comprising vaccines, antibodies, antigens, anticoagulants, hormones, growth factors, enzymes; and of diagnostic molecules such as receptor proteins, allergens, and biomarkers.

In another preferred embodiment, the invention relates to the use of a recombinant algal cell as provided herein for the production of nutritional compounds for human or animal feed selected from the group comprising food or feed proteins, enzymes such as proteases and peptidases, carbohydrases, lipases and esterases.

In yet another aspect, the invention relates to a recombinant protein obtained by carrying out a method according to the invention and to the use thereof, e.g. for pharmaceutical, biomedical, diagnostic, cosmetic, agro-food, environmental and biotechnological applications.

### Description of the figures

**FIGURE 1** illustrates an embodiment of an expression vector (pHR3 plasmid) that can be used in a method of the present invention.
**FIGURE 2** illustrates another embodiment of an expression vector (pSL18 plasmid) that can be used in a method of the present invention.
**FIGURE 3** illustrates the results of an electrophoresis in 1% agarose gel of PCR products (fragment of the 28S rRNA gene) obtained from cells of 2 clones of *S*.
*obliquus* (wild strain) as described in the example 6 of the present invention. Well 1 is a Standard Marker (Smart Ladder, Eurogentec); well 2 is clone 1 of *S*. *obliquus;* well 3 is clone 2 of *S*. *obliquus.*
**FIGURE 4** illustrates another embodiment of an expression vector (pHR3-sscGH1 plasmid) that can be used in a method of the present invention.
**FIGURE 5** illustrates another embodiment of an expression vector (pHR3-cGH3 plasmid) that can be used in a method of the present invention.

### Detailed description of the invention

### Definitions

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

The term "polynucleotide" or "nucleotide sequence" or "nucleic acid molecule" is used broadly herein to mean a sequence of two or more deoxyribonucleotides or ribonucleotides that are linked together by a phosphodiester bond. As such, the terms include RNA and DNA, which can be a gene or a portion thereof, a cDNA, a synthetic polydeoxyribonucleic acid sequence, or the like, and can be single stranded or double stranded, as well as a DNA/RNA hybrid. Furthermore, the terms as used herein include naturally occurring nucleic acid molecules, which can be isolated from a cell, as well as synthetic polynucleotides, which can be prepared, for example, by methods of chemical synthesis or by enzymatic methods such as by the polymerase chain reaction (PCR).

The terms "recombinant nucleic acid" or "recombinant nucleic acid molecule" as used herein generally refer to nucleic acid molecules (such as, *e.g*., DNA, cDNA or RNA molecules) comprising segments generated and/or joined together using recombinant DNA technology, such as for example molecular cloning and nucleic acid amplification. Usually, a recombinant nucleic acid molecule may comprise one or more non-naturally occurring sequences, and/or may comprise segments corresponding to naturally occurring sequences that are not positioned as they would be positioned in a source genome which has not been modified. When a recombinant nucleic acid molecule replicates in the host organism (herein an algal cell) into which it has been introduced, the progeny nucleic acid molecule(s) are also encompassed within the term "recombinant nucleic acid molecule".

In particular embodiments, a recombinant nucleic acid molecule can be stably integrated into the genome of a host organism (herein an algal cell), such as for example integrated at one or more random positions or integrated in a targeted manner, such as, *e.g*., by means of homologous recombination, or the recombinant nucleic acid molecule can be present as or comprised within an extra-chromosomal element, wherein the latter may be auto-replicating.

The term "recombinant protein" or "recombinant polypeptide" or "protein of interest" or "polypeptide of interest" as used herein refers to a polypeptide or protein produced by a host organism (herein an algal cell) through the expression of a recombinant nucleic acid molecule, which has been introduced into said host organism or an ancestor thereof, and which comprises a sequence encoding said polypeptide or protein.

The present invention is not limited in the type or function of recombinant protein that can be produced and secreted in accordance with the present invention. In an embodiment, the recombinant protein may be a heterologous polypeptide, meaning a polypeptide that does not naturally occur in the algal host cell. In another embodiment, the recombinant protein may be a homologous polypeptide, meaning a polypeptide native or naturally occurring in a host cell. In one embodiment, the invention includes algal host cells producing the homologous polypeptide via recombinant DNA technology.

Further, the terms "recombinant" or "transformed" as used herein with reference to algal cells, encompass such algal cells into which a recombinant nucleic acid molecule has been introduced, as well as the recombinant progeny of such cells.

Hence, the term "transformation" encompasses the introduction or transfer of a foreign nucleic acid such as a recombinant nucleic acid into an algal host cell as defined herein. The so-introduced nucleic acid may be preferably maintained throughout the further growth and cell division of said host cell.

By "encoding" is meant that a nucleic acid sequence or part(s) thereof corresponds, by virtue of the genetic code of an organism in question to a particular amino acid sequence, *e.g*., the amino acid sequence of a desired polypeptide or protein. By means of example, nucleic acids "encoding" a particular polypeptide or protein may encompass genomic, hnRNA, pre-mRNA, mRNA, cDNA, recombinant or synthetic nucleic acids. Preferably, a nucleic acid encoding a particular polypeptide or protein may comprise an open reading frame (ORF) encoding said polypeptide or protein. An "open reading frame" or "ORF" refers to a succession of coding nucleotide triplets (codons) starting with a translation initiation codon and closing with a translation termination codon known *per se*, and not containing any internal in-frame translation termination codon, and potentially capable of encoding a polypeptide. Hence, the term may be synonymous with "coding sequence" as used in the art. A coding sequence can be interrupted by introns.

Expression of polypeptides of interest in recombinant microorganisms as taught herein can be achieved through operably linking the nucleic acid sequences or ORF(s) which encode said polypeptides with regulatory sequences allowing for expression in said microorganisms. In this regard, the term "expressing" a polypeptide when referring to a recombinant organism encompasses recombinant organisms capable of expressing the polypeptide, e.g. under suitable culture conditions or upon addition of inducers (e.g. where inducible regulatory sequences are used).

An "operable linkage" is a linkage in which regulatory nucleic acid sequences and sequences sought to be expressed are connected in such a way as to permit said expression. For example, sequences, such as, *e.g*., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

The precise nature of regulatory sequences or elements required for expression may vary between organisms, but typically include a promoter and a transcription terminator, and optionally an enhancer.

Reference to a "promoter" or "enhancer" is to be taken in its broadest context and includes transcriptional regulatory sequences required for accurate transcription initiation and where applicable accurate spatial and/or temporal control of gene expression or its response to, *e.g*., internal or external (*e.g*., exogenous) stimuli. More particularly, "promoter" may depict a region on a nucleic acid molecule, preferably DNA molecule, to which an RNA polymerase binds and initiates transcription. A promoter is preferably, but not necessarily, positioned upstream, *i.e.,* 5', of the sequence the transcription of which it controls.

In embodiments, promoters contemplated herein may be constitutive or inducible.

It shall also be appreciated that the nucleic acids of the invention may encode one or more than one polypeptides of interest. Moreover, where expression of two or more polypeptides is intended, said expression may be from independent expression units, or may be from a single expression unit (*i.e.,* multi-cistronic expression) comprising two or more sequential ORFs controlled by common regulatory elements. By means of example, an expression unit encoding two or more polypeptides may be generated by associating translation initiation codons of the respective ORFs with sequences controlling translation initiation (*e.g*., ribosome entry sequences).

The terms "terminator" or "transcription terminator" refer generally to a sequence element at the end of a transcriptional unit which signals termination of transcription. The sequence of the terminator also contains the polyadenylation signal. For example, a terminator is usually positioned downstream of, *i.e.,* 3' of ORF(s) encoding a polypeptide of interest. For instance, where a recombinant nucleic acid contains two or more ORFs, *e.g*., successively ordered and forming together a multi-cistronic transcription unit, a transcription terminator may be advantageously positioned 3' to the most downstream ORF.

In a preferred embodiment, regulatory sequences controlling the expression of polypeptide(s) as taught herein may be advantageously provided on the recombinant nucleic acid to be used for transforming the present algal cells. However, also contemplated are embodiments in which the recombinant nucleic acid provides for coding sequence(s) lacking one or more regulatory sequences, whereas the required regulatory sequences are provided by the transformed algal cells (such as, *e.g*., wherein the recombinant nucleic acid inserts into a chromosomal region comprising suitable regulatory sequences).

An "expression vector" is a construct that can be used to transform a selected algal host cell and provides for expression of a coding sequence in the selected host. Expression vectors can for instance be cloning vectors, binary vectors or integrating vectors. The invention thus also relates to a vector comprising any of the nucleic acids described herein. Said vector may further comprise regulatory sequences for controlling expression of the nucleic acid in said host cell, as defined above. In an embodiment in the algal systems concerned by this application, the vectors are integrated randomly in nuclear chromosomal DNA.

Expression vectors as applied in the present invention typically also comprise a selectable marker gene. As used herein, the term "selectable marker gene" includes any gene, which confers a phenotype on a cell in which it is expressed to facilitate the identification and/or selection of cells which are transformed with an expression vector of the invention.

With the insight to better show the characteristics of the invention, some preferred embodiments and examples are now described hereafter referring to the enclosed drawings.

### Algal host cells

The present invention is directed to a method for the production of a protein of interest by an algal cell. Algal cells that are used in accordance with the present invention are fresh water microalgae.

The term "fresh water algae" refers to algae that grow in freshwater ecosystems, as opposed to marine algae. Preferably, algal cells that are used in accordance with the present invention are fresh water unicellular green microalgae (i.e. microscopic algae) of the class of the *Chlorophyceae.* In another preferred embodiment, algal cells that are used in accordance with the present invention are of the order of the *Sphaeropleales.*

As indicated above, in view of characteristics of algal cells of this order, for instance presence of a polymer called "sporopollenin-like", which is expected to hinder genetic engineering of this type of algal cells of this order is obvious. Despite this teaching the applicants have established a reproducible method for genetically engineering algal cells of this order.

In a preferred embodiment, said algal cells are selected from but not limited to the group comprising *Scenedesmus, Desmodesmus, Tetradesmus, Sphaeroplea, Tetraedron, Bracteacoccus, Selenastrum, Monoraphidium, Ankistrodesmus, Coelastrum, Pediastrum, Enallax, Scotiellopsis,* and *Sorastrum.* This list of groups is not exhaustive.

In a preferred embodiment, said algal cells are of the genus *Scenedesmus,* and for instance are selected from the group comprising but not limited to *S*. *obliquus, S. acuminatus, S. acutiformis, S. armatus, S. costato-granulatus, S. falcatus, S. producto-capitatus, S. subcapitatus, S. wisconsiensis, S. abundans, S. acutus, S. aldevei, S. arcuatus, S. basiliensis, S. costatus, S. serratus, S. carinatus, S. maximus, S. perforatus, S. obtusus, S. dimorphus, S. ellipticus, S. quadricauda, and S. communis.* The list of *Scenedesmus* species is not exhaustive.

### Expression vectors

In a first method step, an expression vector for transforming the algal cells as defined herein is prepared. Preferred expression vectors for use in the present invention may contain the following elements:
- a bacterial origin of replication and a selective marker;
- at least one selection marker gene that can contain non algal DNA sequences, for instance consisting of a promoter sequence, a coding sequence and a terminator; and
- at least one expression cassette that comprises a protein coding sequence isolated from the same alga or from another organism, this sequence being inserted downstream of a regulated (induced) or constitutive promoter and upstream of a terminator.

In an embodiment said promoter may consist of sequences isolated from for instance algae, plants, Eumycetes, etc. such as but not limited to the following promoter sequences: *psaD,* β*-tubulin, rbcS2, hsp70A, rbcS2-hsp70A* hybrid, *CaMV 35S.* A whole promoter or fragments of it can be used.

Algal cells may express and accumulate a protein of interest in an area selected from the group consisting of nucleus, chloroplast, mitochondria, cytoplasm, periplasmic space, cell membrane, or cell wall, depending of the presence or absence of certain targeting sequences in the expression cassette. In an embodiment, the protein coding sequence can be fused to a nucleotide sequence coding for a secretion signal peptide so as to obtain the secretion of the recombinant protein. This nucleotide sequence can originate from for instance plants, algae, Eumycetes, etc. or it can be a synthetic nucleotide sequence.

The expression cassette can also include one or several introns located between the promoter and the protein coding sequence, or inside the protein coding sequence. These introns can originate from plant or algal genes, or from other eukaryotic genes. The protein coding sequence can also be fused to a nucleotide sequence coding for a tag so as to facilitate identification and/or purification of the recombinant protein. This tag can be located at the carboxy terminus or at the amino terminus of the protein. Examples of tags include but are not limited to a poly-His tag, a Flag-tag, an S-tag, a GST-tag, a GFP-tag, a CBDs-tag, an MBP-tag.

The terminator can originate from bacteria, plants, algae, Eumycetes or other organisms, and can be one selected from the group comprising *rbcS2, psaD, rbcL,* and *psbA.* The list of terminators is not exhaustive.

The protein coding sequence can also be fused to the coding sequence of another protein so as to produce a fusion protein.

Expression vectors according to the invention may also comprise more than one expression cassette each comprising elements as defined above.

Protein coding sequences can be used individually to construct at least one expression vector from the group consisting of genome expression vectors, chloroplast expression vectors, and autonomously replicating expression vectors.

As mentioned above, the expression vectors typically comprise at least one algal selection marker gene. In an embodiment, selective agents for the micro-algae used in the present invention can be antibiotics or herbicides. The selection can also be based on the complementation of an auxotrophic mutation. **Table 1** presents a non exhaustive list of selective markers (selection marker genes) suitable for use in vectors and methods of the present invention.

**TABLE 1: Selective agents and origin of selective marker that can be used in selecting micro-algae that are transformed according to a method of the present invention**

| Type of selective system | Class of selective agent | Selective agent | Origin of marker |
|---|---|---|---|
| Antibiotics | aminoglycosides | paromomycin | *aphVIII* gene from *Streptomyces rimosus* |
| | | neomycin | *nptII* gene from *Escherichia coli* |
| | | hygromycin | *aph7*" or *hph* gene from *Streptomyces hygroscopes*; *hpt* gene from *E. coli* |
| | bleomycins | bleomycin zeocin phleomycin | *ble* gene from *Streptoalloteichus hindustanus* or from *Staphylococcus aureus* |
| Herbicides | amino-phosphonates | glufosinate ammonium glufosinate | *bar* gene from *S*. *hygroscopes*; *pat* gene from *S*. *viridochromogenes* |

The selective agents are added to the culture medium so as to selectively grow the genetically modified micro-algae. The concentration of each of the selective compounds has to be determined previously through assaying sensitivity of the micro-algae. Example 1 for instance illustrates conditions for selecting *Scenedesmus obliquus* cells transformed with a vector containing an *aphVIII, aph7*", *ble* or *bar* selective marker.

There is no particular limitation towards the type of proteins of interest that can be produced in accordance with the present invention.

In an embodiment said protein of interest is a homologous protein originating from the microalgae.

In another embodiment said protein of interest is a heterologous protein originating from an organism other than said microalgae, and for instance originating from an organism from the group comprising humans, animals, plants, micro-organisms, or other algae or microalgae. In an example said protein of interest originates from bacteria, fungi, viruses, plants, humans, mammals, or fishes.

In a embodiment of the present invention, the protein coding sequence may be derived from at least one selected from the group consisting of humans, animals, plants, including algae or microalgae, and micro-organisms, which can be either cloned from any genomes from above-mentioned organisms, artificially synthesized or amplified by polymerase chain reaction (PCR) in vitro.

There is no limitation towards the type of protein of interest that can be produced by a method according to the invention. In an embodiment, said protein of interest is selected from the group comprising vaccines; antibodies; antigens; hormones; enzymes; therapeutic molecules such as e.g. monoclonal antibodies, anticoagulants, hormone peptides, growth factors; diagnostic molecules such as e.g. receptor proteins, allergens, biomarkers; food proteins such as e.g. industrial enzymes like proteases and peptidases, carbohydrases, lipases and esterases, etc.

In an embodiment, the present method permits the production of important proteins/enzymes.

In an example, homologous proteins of e.g. *Scenedesmus* which can in accordance with the present method be produced in high quantities include for instance hydrogenases. These enzymes are involved in hydrogen metabolism in unicellular green algae that can produce hydrogen gas upon illumination. It is noted that the *HydA* gene has been isolated from *Scenedesmus obliquus* (Florin L. et al., 2001. J. Biol. Chem., 276, 6125-6132). Production of hydrogenases by green algae has potential as alternative energy source.

In another example, proteins which can in accordance with the present method be produced in high quantities by genetically modified green algae such as *Scenedesmus* include for instance enzymes such as desaturases and/or elongases involved in the DHA (docosahexaenoic acid) biosynthetic pathway. DHA is an essential component of cell membranes and is vital for proper neurological development in infants. In adults DHA deficiency has been associated with cognitive decline, coronary heart disease, etc. In humans, DHA is not produced and this must be obtained from dietary sources, like marine fish or microalgae (commercial DHAenriched oils).

In another embodiment, the present method permits the modification of the glycosylation pathway of strains of Sphaeropleales to obtain a strain in which the glycosylation pathway of complex proteins is modified so as to produce humanized glycoproteins. In animal cells the mechanism of N-glycan synthesis involves the addition of galactose residues and sialic acids added to the N-glycan. In contrast, in plant and algae, xylose and fucose residues are added without galactose and sialic acid. In accordance with the present method endogenous Golgi glycosyltransferases involved in the addition of xylose and fucose to the N-glycan core, are inhibited (e.g. by mutation, inactivation, etc.) and the method includes the introduction and expression of missing human genes responsible of the addition of galactose (gene coding for β-1,4-galactosyltransferase) and of sialic acid (genes coding for sialic acid synthetase and transporter, and sialyltransferase).

### Transformation

Several methods can be used to introduce genetic material in algal cells. In fact, transformation techniques in step c) of the present method can be any one of the methods for genetic transformation such as without limitation electroporation, biolistic transformation, chemical transformation, agitation with glass beads, lipofection, virusor bacteriophage-mediated transfection, etc..

The genetic material used for transformation consists of a plasmid vector either circular or linear (after enzymatic restriction). During transformation, the vector is integrated (incorporated) into the algal nuclear genome. Integration into the chloroplast or the mitochondrial genome is also possible.

Transformation can be carried out of algal cells, as for instance illustrated in example 3, or of protoplasts made competent by chemical treatment, as for instance illustrated in example 4.

In a particularly preferred embodiment, a method is provided wherein transformation is carried out by means of electroporation on algal cells.

Unexpectedly, although the prior art usually teaches that electroporation should preferably be carried out on protoplasts, i.e. cells of which the cell wall has been completely or partially removed the Applicants have now shown that highly effective transformation can be obtained by electroporation of intact (whole) algal cells, i.e. algal cells that have not been previously treated or digested and that still contain their cell walls. In an example, the present invention permits to obtain transformation yields of *Scenedesmus* whole cells of more than 1% or of more than 2%, and for instance in the range of 0.4 to 2.2% or of 0.7 to 2.2%.

Moreover, surprisingly, the Applicants were able to obtain transformation yields by electroporation in *Scenedesmus* that are much higher than those reported for other genera. For instance, where transformation rates by electroporation of *Chlamydomonas reinhardtii, Dunaliella salina,* and *Chlorella spp.* are comprised up to 0.28%, up to 0.2% and up to 0.007% respectively, the applicants were able to obtain transformation rates for *Scenedesmus obliquus* of more than 2 %.

### Selection of transformants

A next step of the present method involves selection and analysis of transgenic algal cells as defined herein.

After transformation, the transformed algae are typically placed for 12-24 h in the dark for cell wall regeneration. Then the culture is diluted in liquid medium and the cells are spread on solid medium supplemented with an appropriate selective agent at a concentration which is inhibitory to untransformed algae. Agar plates are generally incubated at 18-25°C under light. After 2 to 4 weeks, colonies are scored, harvested and transferred on fresh selective medium.

In one embodiment, to verify the integration of genetic constructs in the genome, the transformants are screened by PCR analysis either on purified genomic DNA. This is for instance illustrated in Example 7.

In another embodiment, to verify the integration of genetic constructs in the genome, the transformants are screened by PCR analysis on DNA quickly isolated from colonies. This is for instance illustrated in Example 6.

More in particular, according to the present invention, a method is provided for screening transformed algal cells, which comprises the steps of:
i) preparing an aqueous extract of transformed algal cells by transferring said transformed algal cells in an amount of water,
ii) optionally incubating said aqueous extract prepared in step i) at a temperature lower than -20°C for at least 1 hour;
iii) incubating the aqueous extract prepared in step i) or in step ii) at a temperature of at least 95°C for at least 5 minutes; and
iv) subjecting an amount of said aqueous extract to a PCR analysis.

In a preferred embodiment, the invention comprises the step of picking up colonies from agar plates and transferring these colonies to an aqueous extract, for instance less than 55 µl, and for instance between 20 and 50 µl.

In another embodiment, said aqueous extract may be subsequently subjected to a cold chock before being heated. In an embodiment, said aqueous extract may be subsequently incubated at a temperature lower than -20°C for at least 1 hour, and for instance at a temperature of -70°C or lower for 1 to 2 hours.

In yet another embodiment, said aqueous extract is incubated at a temperature of at least 95°C for at least 5 minutes; and for instance at a temperature of more than 99°C for 5 to 15 minutes.

It shall be noted that the described method is easy and fast for screening transformed microalgal clones in general and is not restricted in terms of algal species or genera. The method can be applied to many genera/species of microalgae.

The Applicants have developed a screening method for screening and selecting transformed algae cells that is particularly easy and effective, despite the technical prejudices that would refrain a skilled person from developing such method. In fact, one difficulty that is encountered when transforming algal cells is that the number of copies of recombinant nucleic acids that can be integrated in the genome of algal cells is generally low. As a consequence, PCR analysis using DNA directly obtained from algal clones (without growing-up the clones) is less effective as the generated PCR signal will generally be too weak to allow adequate selection of transformed cells. However, despite this technical prejudice, the Applicants have unexpectedly shown that an screening method based on a direct extraction of DNA from picked up clones can be used for adequately selecting transformed cell, when pre-treating extracted DNA with a heat shock, optionally in combination with a cold shock.

### Culturing of transgenic algae for the expression of recombinant proteins

In a next step the selected algal cells are cultured in a suitable culture medium under conditions permitting the production of the recombinant protein.

In an example, to produce recombinant proteins, genetically modified micro-algae are cultivated in liquid medium; in sterile conical flasks or bioreactors; in auto-, mixo- or heterotrophic conditions; under or without agitation; for instance at 18-25°C. Depending on the volume of the final culture, one or more pre-cultures can be carried out to increase the cell density of the inoculum. After a few days to several weeks, the culture is centrifuged to separate cells and supernatant.

Depending on the expression vector, the recombinant protein may be intra- or extracellular. Genetically modified micro-algae may be used as whole cells or as subparts of them; the produced recombinant protein may be either purified to homogeneity or partially purified, using techniques that are known in the art, and that will therefore not be disclosed in detail herein.

### USES

Methods, constructs and recombinant algal cell as defined herein can be used to produce various types of recombinant proteins.

In an embodiment, the invention relates to the use of a recombinant algal cell as defined herein that is obtained or obtainable with by carrying out a method as described herein for the production of a recombinant protein. Recombinant algal cells as defined herein can be used in various applications, including pharmaceutical, biomedical, diagnostic, cosmetic, agro-food, environmental and/or biotechnological applications.

In a preferred embodiment, the invention relates to the use of a recombinant algal cell as provided herein for the production of therapeutic proteins selected from the group comprising vaccines, antibodies, antigens, anticoagulants, hormones, growth factors, enzymes; and of diagnostic molecules, for instance selected from the group comprising receptor proteins, allergens, and biomarkers.

In another preferred embodiment, the invention relates to the use of a recombinant algal cell as provided herein for the production of nutritional compounds for human or animal feed selected from the group comprising food or feed proteins, enzymes for instance selected from the group comprising proteases and peptidases, carbohydrases, lipases and esterases.

Recombinant algal cells according to the invention can be administered or fed to humans or animals, including aquatic organisms (such as fishes). In an example, the algae-based production of recombinant proteins as disclosed permits oral delivery of a vaccine, antibodies, food proteins, etc. Therefore, the present invention also relates to composition, preferably for oral administration, comprising a recombinant algal cell as defined herein.

Recombinant proteins can also be individually useful as agents, i.e. when separated from the algal cell, e.g., in detergents, synthetic transformations, medicaments, cosmetic agents, diagnostic agents and the like. In this context, the present invention also provides a method for production of desired recombinant proteins by culturing algal cells transformed according to this invention followed by isolating the produced desired protein. The desired proteins can be produced in cultures of algae grown under suitable conditions more optimal for growth and recovered by standard protein recovery methods suitable for the desired protein. In yet another aspect, the invention therefore also relates to recombinant proteins obtained by culturing recombinant algal cells according to the invention and to the use thereof, e.g. for pharmaceutical, biomedical, diagnostic, cosmetic, agro-food, environmental and biotechnological applications.

In another aspect, the invention relates to a recombinant algal cell or to a recombinant protein according to the invention for use as a medicament. In another aspect, the invention relates to a recombinant algal cell or to a recombinant protein according to the invention for use as a cosmetic agent. In another aspect, the invention relates to a recombinant algal cell or to a recombinant protein according to the invention for use as a diagnostic agent. In yet another aspect, the invention relates to a recombinant algal cell or to a recombinant protein according to the invention for use as a food or feed composition, e.g. for use as a food supplement or as a food ingredient.

A wide variety of proteins can be produced by recombinant algae according to the invention. For instance, recombinant algal cells according to the invention can be used to produce recombinant proteins selected from the list comprising but not limited to vaccines; antibodies; antigens; hormones; enzymes; therapeutic molecules such as e.g. monoclonal antibodies, anticoagulants, hormone peptides, growth factors; diagnostic molecules such as e.g. receptor proteins, allergens, biomarkers; food proteins such as e.g. industrial enzymes like proteases and peptidases, carbohydrases, lipases and esterases, etc.

One group of proteins which are directly useful are hormones. Illustrative hormones include but are not limited to parathyroid hormone, growth hormone such as e.g. canine growth hormone (cGH), gonadotrophins, (FSH, luteinizing hormone, chorionogonadotrophin, and glycoproteins), insulin, ACTH, somatastatin, prolactin, placental lactogen, melanocyte stimulating hormone, thyrotropin, parathyroid hormone, calcitonin, enkephalin, and angiotensin and the like.

Other proteins of interest include but are not limited to vaccines, serum proteins, fibrinogen, prothrombin, thromboplastin, globulin, e.g., gamma-globulins or antibodies, heparin, antihemophilia protein, oxytocin, albumins, actin, myosin, hemoglobin, ferritin, cytochrome, myoglobin, lacto globulin, histones, avidin, thyroglobulin, interferon, kinins and transcortin and the like.

Another group of proteins of interest includes enzymes. Enzymes may be used for fulfilling a wide variety of functions. These functions include but are not limited to nitrogen fixation, production of amino acids, e.g., polyiodothyronine, particularly thyroxine, vitamins, both water and fat soluble vitamins, antimicrobial drugs, chemotherapeutic agents, e.g., antitumor drugs, polypeptides and proteins e.g. enzymes from apoenzymes and hormones from prohormones, diagnostic reagents, energy producing combinations, e.g., photosynthesis and hydrogen production, prostaglandins, steroids, cardiac glycosides, coenzymes, and the like.

In examples listed below are provided for exemplification purposes only and are not intended to limit the scope of the invention.

### Examples

In the following examples, reference is made to the *Scenedesmus obliquus* strain CCAP 276/3B, which was obtained from the CCAP culture collection (Culture Collection of Algae and Protozoa), Dunstaffnage Marine Laboratory, Oban, Argyll, PA37 1QA, UK.

### Example 1

This example illustrates conditions for selecting *Scenedesmus obliquus* cells that have been transformed with a vector containing an *aphVIII, aph7*", *ble* or *bar* selective marker. The conditions are represented in Table 2.

**Table 2**

| Selective agent | Gene marker | Concentration of selective agent (mg/l) |
|---|---|---|
| Paromomycin | *aphVIII* | 10-15 |
| Hygromycin | *aph7"* | 20 |
| Phleomycin | *ble* | 30 |
| Ammonium glufosinate | *bar* | 1000-1500 |

### Example 2

Example 2 illustrates two embodiments of expression vectors, i.e. the pHR3 and pSL18 vectors that can be used in a method of the present invention.

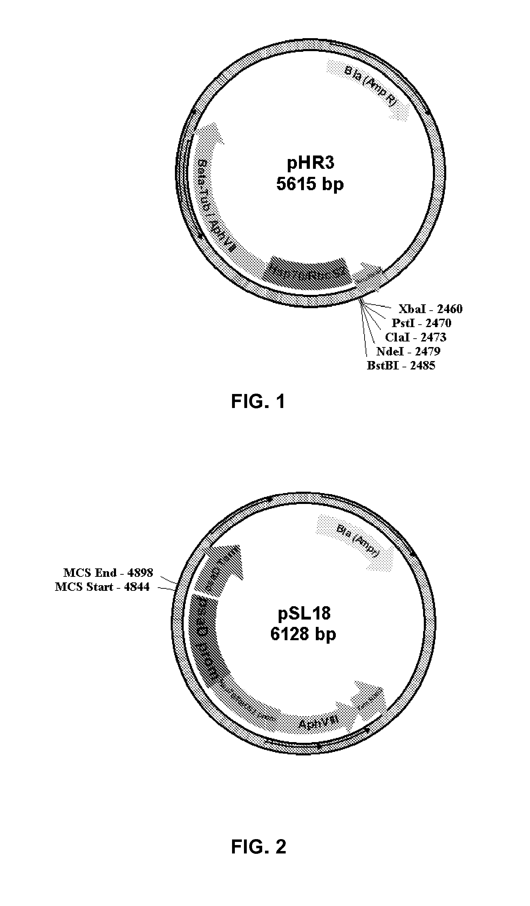

The pHR3 vector (5615 bp) was used for expressing proteins in *S*. *obliquus.* It consisted mainly in the following elements (see **FIG. 1**): a bacterial origin of replication and a bacterial selective marker (*bla* gene); an expression cassette containing the *Chlamydomonas reinhardtii* β2-tubulin promoter, the coding sequence of the *S*. *hygroscopicus aph7*" gene conferring hygromycin resistance to transformed algae, and the *C*. *reinhardtii rbcS2* terminator; a protein expression cassette containing the *hsp70A-rbcS2* hybrid promoter and the *C*. *reinhardtii rbcS2* terminator, as well as a multiple cloning site bearing among others the *Bst*BI, *Nde*I, *Cla*I, *Pst*I and *Xba*I restriction sites used for cloning a sequence to be expressed.

The pSL18 vector (6128 bp) (N. Fischer & J.-D. Rochaix (2001). Mol Gent Genomics 265 :888-894) was also used for recombinant protein production in *S*. *obliquus.* This plasmid bore the following elements **(****FIG. 2****):** a bacterial origin of replication and a bacterial selective marker (bla gene); a gene cassette made of the *C*. *reinhardtii hsp70A-rbcS2* hybrid promoter, the *S*. *rimosus aphVIII* coding sequence conferring paromomycin resistance to transformed algae and the *C*. *reinhardtii rbcS2* terminator; a protein expression cassette containing the *C*. *reinhardtii psaD* promoter and *psaD* terminator separated by a multiple cloning site bearing among others the *Nde*I, *Cla*I, *Ec*oRV, *Eco*RI, *Pst*I*, Spe*I and *Xba*I restriction sites used for cloning a sequence to be expressed.

### Example 3

This example illustrates the preparation of *S*. *obliquus* competent cells and transformation by electroporation.

A 20 ml volume of EG or EG:JM medium in a 100 ml conical flask was inoculated with algae grown and kept on EG or EG:JM agar medium. EG and EG:JM medium are well known in the art and will not be disclosed in detail herein.

Algae were grown with orbital agitation (80-100 rpm) at 20-25°C under light (34 µmol m⁻² s⁻¹; 12 h day, 12 h night) for a period of 72-96 h (corresponding to the exponential growth phase for *S*. *obliquus*). Cell density was monitored by counting cell number on a Bürker cell. At the end of the period cell density was 10⁷ to 2.10⁷ cells/ml. Cells were collected by low speed centrifugation for 5 to 10 minutes. They were washed with 50 ml of 7 mM Hepes, 252 mM glucose, pH 7.0. After centrifugation, washing was repeated. Cells were then suspended in sterile distilled water at a final density of 1 to 2.10⁸ cells/ml and kept on ice for 1-2 h. 80 µl of cell suspension were mixed with a maximum volume of 15 µl containing 0.5 to 5 µg of plasmid DNA. Plasmids were prepared with the Nucleobond AX kit (Machery-Nagel) following the manufacturer instructions. They were checked by restriction and agarose gel electrophoresis. DNA concentration was assayed either by measuring optical density at 260 nm, or by comparing UV fluorescence in agarose gel with that of a DNA standard. Plasmids were used as circular or linear DNA. In the latter case either the whole plasmid was used after restriction, or the expression cassettes were excised and purified before use. In this latter case the excised DNA fragment consisted either of sequences belonging only to the expression cassettes, or of slightly longer DNA fragment so as to avoid degradation by exonucleases during the transformation process. Herring sperm DNA can also be added (plasmid DNA - herring DNA 1:2) to compete for nuclease activities and therefore protect plasmid DNA. Electroporation was carried out in the following conditions: electric field 1800 V/cm, shunt 200 Ω, capacitance 25 µF. Three controls were included in each transformation experiment: non electroporated competent cells, competent cells electroporated without DNA, and competent cells electroporated with herring sperm DNA only.

### Example 4

This example illustrates the preparation and transformation of *S*. *obliquus* protoplasts.

Protoplast preparation implies partial removal of cell wall material. This can allow DNA penetration into the cell. Cell wall hydrolysis is brought about by a mixture of hydrolytic enzymes (cellulases, pectinases, macerase ...). Cell wall digestions can be monitored under the microscope. Two procedures were used to generate protoplasts. In the first one the digestion step was applied on cells from a 50 ml culture (carried out as in example 3) at a final density of 10⁸ cells/ml. After washing the cells in 25 mM phosphate buffer pH 6.0, they were centrifuged, suspended in 5 ml of the same buffer and incubated in a mix of 4% cellulase, 2% macerase and 50 units driselase for 16 h in the dark with gentle agitation. In the second procedure the cells grown as above were first washed in culture medium (EG or EG:JM) containing 1 M mannitol and 1 M sorbitol. The cells were then incubated for 8 h at 37°C in a mix of 4% cellulase, 2% macerase, 4% hemicellulase and 2% driselase. An additional 4% hemicellulase and 2% driselase were then added and incubation was continued for 16 h at 37°C. In the two procedures, after enzymatic digestion, the cells were washed in culture medium (EG or EG:JM) containing 1 M mannitol and 1 M sorbitol. The protoplasts were used for transformation either by electroporation, or by chemical treatment. In the former, conditions were identical to those described in example 3. In the latter, the following steps were carried out. The protoplasts were centrifuged and washed in culture medium (EG or EG:JM) containing 0.6 M mannitol and 0.6 M sorbitol. They were suspended in the same solution containing 50 mM CaCl₂. An amount of 0.5 to 5 µg of plasmid DNA (circular or linear) was added to a volume of about 400 µl of protoplast suspension. Herring sperm DNA can also be added as previously described. The mix was incubated for 15 minutes at room temperature. Then 200 µl of 40% PEG, 0.8 M NaCl, 50 mM CaCl2 were added and the mix was incubated for 30 minutes at room temperature with gentle agitation. After addition of 600 µl of culture medium (EG or EG:JM) containing 0.6 M sorbitol, 0.6 M mannitol, 1% yeast extract and 1% glucose, the cells were incubated for 12 h at room temperature so as to allow cell wall regeneration. The cells were then treated as after transformation by electroporation (see example 5).

### Example 5

This example illustrates a selection of transformants of *S*. *obliquus* after electroporation.

After electroporation, the algal cells were kept on ice for 5 min and suspended in EG or EG:JM medium (5 ml) containing mannitol (1 M) and sorbitol (1 M). The cells were placed for 12-24 h in the dark without agitation. They were then diluted in EG or EG:JM medium to obtain a cell density of ∼ 0.8-1.0.10³ cells/ml. Volumes of 100 µl were spread on EG agar plates supplemented or not with the selective agent. Cultures were incubated at 20-25°C under light (34-40 µmol photons.m⁻².s⁻¹; 12 h day, 12 h night). After 2-4 weeks, each transformed colony was picked up and transferred on fresh EG or EG:JM solid medium with or without the selective agent.

### Example 6

This example illustrates a method for screening of *S*. *obliquus* transformants by PCR on DNA quickly isolated from colonies.

The colonies were picked from agar plates with sterile toothpicks and transferred to 20-50 µl of water. The mix was incubated at 100°C for 5-15 min if necessary after a treatment at -70°C for 1-2 h. The heat treatment could also be carried out in a thermocycler at 99.9°C for 15 min. A volume of 1-5 µl of the mixture was used for PCR.

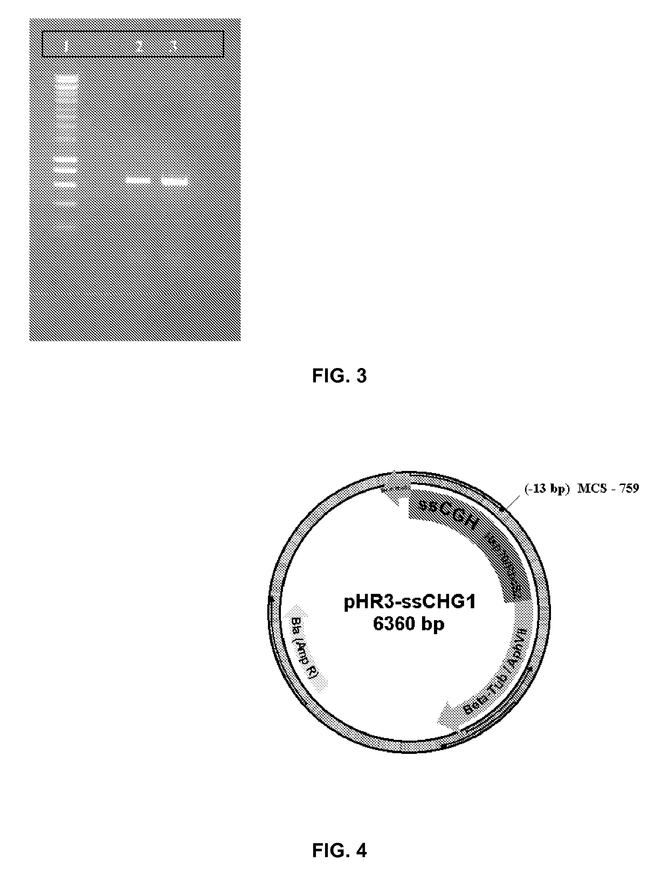

The described method was validated as follows. Two specific oligonucleotide primers were designed at both ends of the endogenous 28S rRNA gene of *S*. *obliquus* to amplify a 600 bp fragment. Each sample consisted of 1-5 µl of the extract (prepared as described above) as template DNA in a final PCR reaction volume of 10-15 µl. PCR were carried out in the following cycling conditions: a single cycle of 15 min at 94°C ; 30 cycles of 45 s at 94°C, 45 s at 55°C and 45 s at 72°C ; 7 min at 72°C before shifting to 4°C. PCR products were analyzed by electrophoresis in 1% agarose gels stained with the fluorescent dye ethidium bromide to visualize DNA bands under UV light. A DNA molecular marker (SmartLadder, Eurogentec) consisting of DNA fragments of known molecular sizes was loaded into one well of the gel and used as a standard to determine the size of the PCR products. The analysis of PCR products obtained by the described method revealed a single 600 bp PCR band **(****FIG. 3****)** which was consistent with the expected result.

The method has also been validated using another gene, the *HydA gene,* which is a low copy (single copy) gene. Results obtained for this gene are believed to represent results that are obtained when integrating a heterologous gene (generally integration of a single copy). The same conditions as explained for the introduction of the 28S rRNA gene were applied for the HydA gene and similar results were obtained.

### Example 7

This example illustrates a method for screening of *S*. *obliquus* tranformants by PCR on genomic DNA. The genomic DNA isolation was modified from Dawson et al. (1997. Curr. Microbiol. 35 :356-62.). Each clone was picked from agar plate with a sterile toothpick and suspended in 2-4 ml of EG or EG:JM medium. The culture was grown with orbital agitation (80-100 rpm) at 20-25°C under light (34-40 µmol photons.m⁻².s⁻¹; 12 h day, 12 h night) for a period of 48-72 h. The preculture was used to inoculate a 20-25 ml volume of EG or EG:JM medium in a 100 ml conical flask. Cells were grown in the same conditions as for the preculture. After a period of 5-7 days, cells were collected by centrifugation and the pellet suspended in 500 µl of CTAB buffer (54 mM CTAB, 0.25 mM Tris [pH 8.0], 1.4 M NaCl, 10 mM EDTA and 2% β-mercaptoethanol). The mix was incubated at 65°C for 2 h and shaken every 15 min. After incubation, an equal volume of phenol:chloroform was added and the whole was mixed by inversion. The aqueous phase was recovered after centrifugation at 13,000 g for 20 min. The extraction was repeated until the aqueous phase was no longer cloudy. The genomic DNA was precipitated with 0.8 volume of isopropanol and 0.1 vol of 3 M sodium acetate, centrifuged at 13,000 g for 30 min, washed with 70% ethanol, centrifuged (13,000 g for 15 min), dried and suspended in 20 µl of water or 10 mM Tris buffer [pH 7.0] containing 0.1 mM EDTA. RNase treatment was carried out by adding 0.5-1 µl of RNase (10 mg/ml) and incubating the mixture at 37°C for 1 h. RNase was then inactivated by heat treatment at 65°C for 15 min. A volume of 5 µl of genomic DNA was used for PCR reaction. The method of genomic DNA isolation was validated in the same conditions as described in the example 6.

The genomic DNA isolation was also carried out with the ZR Solid Microbe DNA kit (Zymo Research) following the manufacturer instructions and the method was validated as described in the example 6.

### Example 8

This example describes embodiments of expression vectors used for expressing canine growth hormone in micro-algae. More in particular, in this example the pHR3-sscGH1 and pHR3-cGH3 plasmids were used for expressing canine growth hormone in micro-algae.

Canine growth hormone, cGH, is a protein produced by the anterior pituitary gland and the mammary glands. The amino-acid and nucleic sequences are available on NCBI, accession AAF21502. Two cDNA sequences were published: one corresponding to cDNA obtained from canine pituitary mRNA (see gene bank ID: CAA80601), and the second one from canine mammary mRNA (see gene bank ID: AAD43366). Both cDNA consisted of 651 bp and are identical except for 2 codons located at the beginning of the sequence (Glycine/Serine and Threonine/Asparagine). The mature protein is 216 amino acids long and is a soluble protein. The cDNA used here corresponds to the canine mammary gland mRNA. The proportion of each nucleotide in the cDNA sequence is: (A) 18.89, (T) 18.59, (C) 33.33 and (G) 29.19. The GC content is 62.52% corresponding to the overall CG content (65%) observed in algal gene sequences. The frequency of XXC/G codons is 86.65% which is close to the mean percentage (90%) of algal nuclear genes. Nevertheless cDNA sequence codons were optimized to efficiently express the mammalian cDNA in micro-algae. Codon usage was chosen to reflect the codon bias of 3 micro-algal species: *S*. *obliquus, Chlorella vulgaris* and *Dunaliella salina.* Furthermore a His-tag (6 CAT) is added at the N- or C-terminus of the protein so as to simplify the identification and/or the purification of the recombinant expressed protein. In the case of the pHR3-sscGH1 plasmid, a signal leader sequence consisting of a synthetic consensus eukaryotic signal sequence is added to the N-terminus of the protein for driving protein secretion. The cGH coding sequence bears at the 5' and 3' ends the following restriction sites to facilitate the cloning: *Cla*I upstream of the signal sequence, *PstI* between the signal sequence and the N-terminus tag sequence, and *Xba*I at the 3' end of the coding sequence.

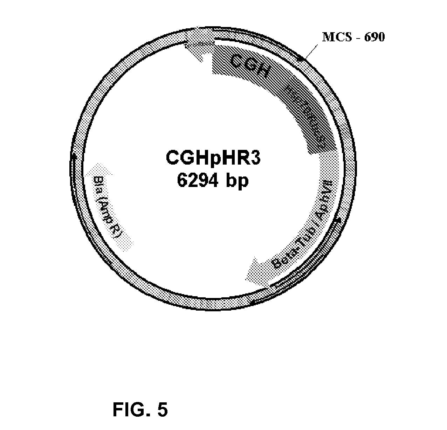

The cDNA coding for cGH was cloned into pHR3 (5615 bp) as follows: the insert consisting of cGH and signal sequence was inserted into *Cla*l/*Xba*l to obtain the pHR3-sscGH1 plasmid (6360 bp; **FIG. 4****);** the cGH sequence alone was inserted into *Pst*l*lXba*l to obtain the pHR3-cGH3 plasmid (6294 bp; **FIG. 5****).**

As studies of transgenes in higher eukaryotes have demonstrated a positive role for introns in gene expression (Lumbreras et al. 1998. The Plant Journal 14(4) :441-47), the first intron of *RBCS2* gene was inserted immediately upstream of the start codon of sscGH sequence (pHR3-sscGH2 plasmid) or cGH sequence (pHR3-cGH4 plasmid).

### Example 9

This example describes the construction of pSL18-sscGH1 and pSL18-cGH3 plasmids for expression of canine growth hormone in micro-algae.

The cDNA of cGH (described in example 8) was cloned into pSL18 plasmid (6128 bp) as follows: the insert consisting of cGH and signal sequence was inserted into *Cla*l*lXba*l to obtain the pSL18-sscGH1 plasmid; the cGH sequence alone was inserted into *Pst*l*lXba*l to obtain the pSL18-cGH3 plasmid. To enhance gene expression the first intron of *RBCS2* gene can be inserted immediately upstream of the start codon of sscGH sequence (pSL18-sscGH2 plasmid) or cGH sequence (pSL18-cGH4 plasmid).

### Example 10

### Discloses the transformation of S. obliquus with a pSL18-sscGH1 plasmid, screening and analysis of transformed colonies

*S. obliquus* electrocompetent cells and electroporation conditions are described in example 3. The pSL18-sscGH1 plasmid is described in example 9. The plasmid was used as circular or linear DNA. In the latter case either the whole plasmid was used after *Kpn*l restriction, or the expression cassette consisting of a 5550 bp fragment obtained by restriction with *Vsp*l was used. After transformation, cells were plated on EG or EG:JM medium supplemented with 10 mg/l of paromomycin. Cultures were incubated at 20-25°C under light (34-40 µmol photons.m⁻².s⁻¹; 12 h day, 12 h night). After 2-4 weeks, the colonies were picked with sterile toothpicks and transferred on fresh EG or EG:JM medium supplemented with 10 and 15 mg/l of paromomycin; thereafter they were subcultured every 2-3 weeks. The colonies picked from selective medium were analyzed to verify the presence of the cGH cassette in algal genomic DNA. Firstly cells were analyzed by PCR on DNA quickly isolated from colonies as described in the example 6. A total of 260 colonies picked from selective medium were analyzed. Each picked clone was suspended in a microtube containing 20 µl of sterile water. A volume of 2-5 µl of the mix was heated in a thermocycler at 99.9°C for 15 min and then used for PCR. Oligonucleotide primers were designed at the 5' and 3' ends of the cGH cDNA. PCR were carried out in the following cycling conditions: a single cycle of 15 min at 95°C; 30 cycles of 45 s at 94°C, 45 s at 68°C and 45 s at 72°C; 7 min at 72°C before shifting to 4°C. PCR products were analyzed by electrophoresis in 1% agarose gels stained with the fluorescent dye ethidium bromide to visualize DNA bands under UV light. A DNA molecular marker (SmartLadder, Eurogentec) was used as a standard to determine the size of the PCR products. The size of PCR products corresponding to the amplified cGH fragment was -780 bp. A total of 73 clones were PCR positive suggesting that they have integrated the heterologous cGH DNA: 20 clones obtained by integration of the circular plasmid and 53 clones obtained by integration of the 5550 bp expression cassette (*Vsp*l restriction). PCR analysis were repeated several times after successive subcultures to verify the stability of both integrated exogenous DNA and transgenic lines. PCR analyses were also carried out on genomic DNA isolated from transgenic clones as described in the example 7. Oligonucleotide primers and PCR conditions were identical to those described above for PCR on DNA quickly isolated from colonies. PCR results showed that more than 50% of strains are stable after 8 months.

### Example 11

### In this example the expression of recombinant cGH by transgenic S. obliquus

Transformants obtained by integration of pSL18-sscGH1 and selected as described in the example 10 were analyzed for cGH expression. The recombinant protein was identified with antibodies raised against either histidine-tag or pig growth hormone. Mature canine and pig GH are 98% identical as demonstrated by Ascacio-Martinez and Barrera-Saldana (1994. Gene 143 :277-80).

A transgenic clone was picked from an agar plate (EG or EG:JM medium) and used to inoculate a 3 ml volume of EG or EG:JM medium in a 15 ml tube. Culture was grown with orbital agitation (80-100 rpm) at 20-25°C under light (34-40 µmol photons.m⁻².s⁻¹; 12 h day, 12 h night) for a period of 2-4 days. A 22 ml volume of EG or EG:JM in a conical flask was inoculated with the 3 ml volume of preculture. The culture was grown for a period of 5-7 days in the same conditions as described above. At the end of the period an aliquot of the culture was harvested to verify by PCR (see method described in the example 6) the presence of the heterologous gene in algal genome. The rest of the culture was centrifuged and the pellet and the supernatant were collected separately. 1 mM PMSF was added to the supernatant to inhibit protease activity. The supernatant was dialyzed (MWCO 6-8,000) against water for 24-48 h and then concentrated by tangential ultrafiltration on a micro-concentrator (Vivaspin, Sartorius; 10,000 cut off) until a final concentration factor of 150 to 300 times. The cell pellet was suspended in 1 ml of Tris-EDTA buffer pH 7.4 containing 1 mM PMSF. An equal volume of glass beads was added and the mix was vortexed for 6 x 1 min alternating with incubation for 6 x 1 min on ice. The intracellular extract was collected by centrifugation and then dialyzed against water for 24-48 h. The extracted proteins were analyzed by SDS-PAGE and western blot using antibodies raised against either histidine-tag or pig growth hormone. An immunoreactive band with molecular weight of - 25-30 kDa (which is in agreement with the expected molecular mass of cGH) was observed in some transformants, either in extracellular or in intracellular compartment.

### Example 12

The present invention also permits to isolate hypersecretion mutants among transformants. This can be done either by the screening transformants and by analyzing the production of the recombinant protein. This can also be done by using another type of genetic construction, for example a fusion between the gene encoding the heterologous protein and the selection marker gene. In this case, the screening of transformants producing high amounts of the protein is carried out on media containing high concentrations of the selection agent. An example is the use of the *Sh ble* gene as selection marker.

In an example, a plasmid pHR3-*Sh ble* is prepared. *Scenedesmus* is transformed with this plasmid by applying techniques as described above, and transformants are obtained, selected and analysed. In a next step a plasmid containing a fusion ble:cGH is constructed, and microalgae, e.g. *Scenedesmus,* is transformed with this plasmid by applying techniques as described above. Transformants are obtained, selected and analysed, e.g. by growing them on media containing high concentrations of the selection agent, and by identifying transformants producing high amounts of the protein (hyperproducers).

## Claims

1. Method for the production of a recombinant protein by an algal cell, wherein said algal cell is of the order of *Sphaeropleales,* capable of producing said recombinant protein, comprising the steps of:
a) preparing an expression vector comprising a recombinant nucleic acid comprising a promoter operably linked to at least one protein coding sequence encoding said recombinant protein,
b) transforming said algal cell with said expression vector;
c) selecting transformed algal cells;
d) culturing said selected algal cell in a suitable culture medium under conditions permitting the production of said recombinant protein,
e) recovering said recombinant protein from said culture medium, and
f) optionally purifying said recovered protein.

2. Method according to claim 1, wherein said algal cell is from the genus *Scenedesmus.*

3. Method according to claim 1 or 2, wherein said algal cell is *Scenedesmus obliquus.*

4. Method according to any of claims 1 to 3, wherein said recombinant protein is a heterologous protein originating from an organism other than said algal cell and preferably from an organism selected from the group comprising humans, animals, plants, micro-organisms, and other algae or microalgae.

5. Method according to any of claims 1 to 4, wherein said recombinant protein is selected from the group comprising vaccines, antibodies, antigens, hormones, enzymes, therapeutic molecules, diagnostic molecules, and food proteins.

6. Method according to any of claims 1 to 5, wherein said expression vector further comprises a selection marker gene operably linked to a promoter.

7. Method according to any of claims 1 to 6, wherein said protein coding sequence is fused to a nucleotide sequence coding for a secretion signal peptide.

8. Method according to any of claims 1 to 7, wherein said protein coding sequence is fused to a nucleotide sequence coding for a tag.

9. Method according to any of claims 1 to 8, wherein said recombinant nucleic acid includes one or more introns located between the promoter and the protein coding sequence, or located inside the protein coding sequence.

10. Method according to any of claims 1 to 9, wherein transformation is done by electroporation of said algal cell.

11. Method according to any of claims 1 to 10 wherein step c) of selecting transformed algal cells comprises the steps of:
i) preparing an aqueous extract of transformed algal cells by transferring said transformed algal cells in water,
ii) optionally incubating said aqueous extract prepared in step i) at a temperature lower than -20°C for at least 1 hour;
iii) incubating the aqueous extract prepared in step i) or in step ii) at a temperature of at least 95°C for at least 5 minutes; and
iv) subjecting an amount of said aqueous extract to a PCR analysis.

12. Recombinant algal cell, wherein said algal cell is of the order of *Sphaeropleales* which is transformed with an expression vector as defined in any of claims 1 and 4 to 9.

13. Recombinant algal cell according to claim 12, wherein said algal cell is as defined in claim 2 or 3.

14. Recombinant algal cell according to claim 12 or 13 obtainable by carrying out a method according to any of claims 1 to 11.

15. Use of a recombinant algal cell according to any of claims 12 to 14 for the production of a recombinant protein, preferably a recombinant protein as defined in claim 4 or 5, suitable for being used in a pharmaceutical, biomedical, diagnostic, cosmetic, agro-food, environmental and/or biotechnological application.

16. Use according to claim 15 of a recombinant algal cell for the production of therapeutic proteins selected from the group comprising vaccines, antibodies, antigens, anticoagulants, hormones, growth factors, enzymes; and of diagnostic molecules such as receptor proteins, allergens, and biomarkers.

17. Use according to claim 15 of a recombinant algal cell for the production of nutritional compounds for human or animal feed selected from the group comprising food or feed proteins, enzymes such as proteases and peptidases, carbohydrases, lipases and esterases.
